# EUROPEAN PATENT APPLICATION

(11) **EP 3 919 606 A1**
(43) Date of publication of application: **08.12.2021**
(21) Application number: 20020256.2
(22) Date of filing: 01.06.2020
(51) Int. Cl.: C12M 1/00, C12M 1/107

(54) **CONVERSION OF ALGAE TO BIOMETHANE**

(71) Applicant: Algae & Algae Technologies Ltd, West Wickham, Kent BR4 0NF (GB)
(72) Inventor: The designation of the inventor has not yet been filed

(57) **Abstract**

**TECHNICAL FIELD**

THE INVENTION PERTAINS TO HIGH YIELD CULTIVATION OF MICROALGAE BIOMASS IN OUTDOOR RACEWAYS THROUGH CAREFUL CONTROL OF ALL GROWTH PARAMETERS AND THE ADJUSTMENT OF THE ALGAE INTERNAL COMPOSITION IN ORDER TO OBTAIN A SUPERIOR VOLUME OF METHANE PER UNIT OF BIOMASS THROUGH ANAEROBIC DIGESTION.

A SELECTED SPECIES OF MICROALGAE IS SEEDED IN COVERED RACEWAYS AND GROWN UNDER STRICTLY CONTROLLED CONDITIONS OF NUTRIENT AND MICRONUTRIENT ADDITION, CO2 ADDITION, ARTIFICIAL LIGHT OF SPECIFIC SPECTRUM FOR THE ALGAL SPECIES AND THE APPLICATION OF A PULSED MAGNETIC FIELD. THE RACEWAY SOLUTION IS CONSTANTLY AGITATED TO ENSURE SUNLIGHT REACHES ALL OF THE MICROALGAE IN THE RACEWAY. THE OBJECTIVE OF CONTROLLING ALL GROWTH PARAMETERS IS TO MAXIMIZE THE CREATION OF BIOMASS BY PROVIDING, IN A TIMELY FASHION, THE ELEMENTS NEEDED BY THE MICROALGAE AS IDENTIFIED IN LABORATORY STUDIES.

THE PULSED MAGNETIC FIELD IMPROVES THE GROWTH RATE OF THE ALGAE WHILE MAINTAINING A GOOD CELL DENSITY ( STIMULATION OF GROWTH AND METABOLIC CASCADES) BY CONTROLLING BIOCHEMICAL PATHWAYS. IN ADDITION THE PHOTOTROPIC CELLULAR COMMUNICATION IS ENHANCED LEADING TO A HIGHER CONTENT OF LIGHT HARVESTING PRIMARY AND ACCESSORY PIGMENTS. THIS RESULTS IN AN IMPORTANT INCREASE IN LIPID CONTENT OF THE ALGAE BIOMASS WHICH IN TURN YIELDS A HIGHER PRODUCTION OF METHANE COMPARED TO THE CARBOHYDRATE AND PROTEIN CONTENTS IN THE BIOMASS.

THE PROCESS RECYCLES THE MAXIMUM QUANTITY OF WATER POSSIBLE AS WELL AS CAPTURING ALL THE CO2 REMOVED FROM THE SCRUBBING OF THE BIOGAS AND RECYCLING IT TO THE RACEWAYS TO PROMOTE ALGAE GROWTH..

## Description

MICROALGAE IS GROWN IN OUTDOOR ENCLOSED RACEWAYS (A1). THESE RACEWAYS ARE ARRANGED IN A PARALLEL GROUPING SO THAT EACH IS INDEPENDENT AND ONE CAN BE TAKEN OUT OF PRODUCTION FOR MAINTENANCE AND REPAIR WITHOUT AFFECTING PRODUCTION IN THE OTHER RACEWAYS.

THE MICROALGAE SEEDED TO THE PONDS IS NORMALLY A MIXTURE OF STRAINS FROM A SINGLE SPECIES TESTED AND OPTIMIZED FOR LOCAL CLIMATIC CONDITIONS.

FOR EACH GROUP OF PONDS, A NUTRIENT MIXING TANK (A2) IS PROVIDED TO PUT INTO SOLUTION ALL NUTRIENTS AND MICRONUTRIENTS BEFORE ADDITION BY METERED PUMPS TO THE INDIVIDUAL RACEWAYS (B1). THE RACEWAYS CONTAIN ONE OR MORE MOTORIZED PADDLEWHEELS (B2) TO KEEP THE ALGAE SOLUTION IN CIRCULATION TO AVOID SEDIMENTATION AND TO PROMOTE MIXING OF NUTRIENTS. AIR IS BUBBLED (B3) INTO THE BOTTOM OF THE RACEWAYS TO AVOID STRATIFICATION INTO LAYERS OF THE ALGAE GROWTH AND ALSO TO CIRCULATE VERTICALLY THE ALGAE SO THAT LIGHT EQUALLY REACHES ALL THE ALGAE PRESENT. CO2 IS INTRODUCED (B4) TO THE RACEWAYS IN NANO AND MICRO BUBBLES TO SUPPLEMENT THE CO2 ADDED FROM THE AIR. THIS CO2 WAS RECOVERED FROM THE SCRUBBING OF THE BIOGAS (A13). THE DENSITY OF THE ALGAE SOLUTION AND WATER PH. ARE MEASURED CONTINUOUSLY(B5).

ABOVE A SECTION OF THE RACEWAY IS SITUATED A LARGE LED PANEL (B6) WITH A PRECISE RATIO OF WHITE, BLUE, AND RED ELEMENTS WHICH IS ACTUATED WHEN SUNLIGHT IS INSUFFICIENT AND DURING NIGHTTIME TO STIMULATE GROWTH.

EVERY RACEWAY CONTAINS A SPECIALLY FABRICATED CONTROLLED MAGNETIC FIELD ENCLOSURE (B7) WHICH PRODUCES A PMF (PULSATING MAGNETIC FIELD). THE ALGAE IN THE RACEWAY CIRCULATES CONTINUOUSLY THROUGH THIS FIELD WHICH STIMULATES THE ALGAE GROWTH AND ENHANCES THE PHOTOTROPIC ABILITY OF THE ALGAE. THE INTENSITY AND DURATION OF THE FIELD IS TUNED TO THE ALGAE SPECIES BEING CULTIVATED.

WHEN THE ALGAE IN THE RACEWAYS REACHES A CONCENTRATION, USUALLY BETWEEN 6-12 GRAMS PER LITER (GPL) SUCH THAT THE GROWTH RATE BEGINS TO SLOW THEN IT IS HARVESTED BY PUMPING OUT 40-60% OF THE RACEWAY VOLUME TO A THICKENER (A3). DURING THE PUMPING A POLYELECTROLYTE IS ADDED TO THE PUMPED SOLUTION TO ACCELERATE SEDIMENTATION.

SEDIMENTATION IS VERY RAPID AND IN 30-60 MINUTES THE THICKENED ALGAE CAN BE PUMPED TO A FILTERING STATION (A4) WHILE THE THICKENER OVERFLOW IS RETURNED TO THE RACEWAYS. BY CONNECTING A NUMBER OF RACEWAYS TO A CENTRALIZED THICKENER THE OPERATION IS NEARLY CONTINUOUS.

THE THICKENED MICROALGAE IS DEWATERED BY A SERIES OF CYCLONES, AND BELT FILTERS. THE RESULTING FILTER CAKE IS 10-15 % SOLIDS CONTENT. THE WATER REMOVED DURING FILTERING IS RETURNED TO THE RACEWAYS WHILE THE ALGAE FILTER CAKE IS CONVEYED TO AN ALGAE CONDITIONING STATION (A5).

THE ALGAE CONDITIONER APPLIES A THERMAL SHOCK AND A VACUUM THROUGH CAVITATION TO RUPTURE THE WALLS OF THE CELLS TO ENABLE A MORE RAPID ATTACK OF ENZYMES AND BACTERIA DURING THE SUBSEQUENT ANAEROBIC DIGESTION (A6).

THE CONDITIONED ALGAE IS THEN CONVEYED INTO ONE OF THE ANAEROBIC DIGESTION TANKS WHICH ARE CONTINUOUSLY STIRRED. THE FERMENTATION AND METHANIZATION OF THE LIPIDS, CARBOHYDRATES AND PROTEINS COMPOSING THE ALGAE TAKES PLACE UNDER MESOPHILIC CONDITIONS AT TEMPERATURES IN THE RANGE OF 32-42°C THE RESIDENCE TIME IN THE TANK IS 10-20 DAYS, BASED ON OPTIMAL BIOGAS YIELD. FOR SOME MICROALGAE SPECIES, THE OPTIMAL RESIDENCE TIME MAY BE DIFFERENT. DURING THIS PROCESS ABOUT 85% OF THE ALGAE BIOMASS IS CONVERTED TO BIOGAS WHICH HAS A TYPICAL COMPOSITION OF 60-70% METHANE AND 25-30 % CARBON DIOXIDE WITH TRACES OF OXYGEN, NITROGEN AND HYDROGEN SULPHIDE. THIS BIOGAS IS ALSO HUMID CONTAINING 3-5%H2O.

FROM THE BOTTOM OF THE ANAEROBIC DIGESTION TANK THE REMAINING WATER AND UNCONVERTED SOLIDS ARE REMOVED. THIS DIGESTATE IS FILTERED (A8) AND THE WATER IS TREATED (A9) BEFORE DISPOSAL AS IT CONTAINS TOO MANY ENZYMES AND BACTERIA TO BE RECYCLED. THE DIGESTATE FILTER CAKE IS TREATED (A10) AND THEN SOLD AS A SOIL CONDITIONER.

THE BIOGAS GENERATED IN THE AD TANKS IS COLLECTED IN THE GAS DOME (A7) FROM WHERE IT IS CONVEYED TO A SERIES OF GAS CLEANING STAGES. THE H2S IS REMOVED BY AN AMINE OR SIMILAR ADSORPTION STEP (A11). THEN THE HUMIDITY OF THE BIOGAS IS REMOVED BY COOLING (A12). THEREAFTER MOLECULAR SIEVES (A13) SEPARATE THE METHANE FROM THE CO2, OXYGEN AND NITROGEN WHICH ARE RECYCLED TO THE RACEWAYS. THE RESULTING PURIFIED BIO -METHANE PASSES TO A GAS COMPRESSION STATION (A14) WHERE IT IS COMPRESSED TO 65-75 BAR BEFORE BEING SAMPLED, MEASURED AND FED INTO THE GAS PIPELINE SYSTEM OR TO A LIQUEFACTION STATION.

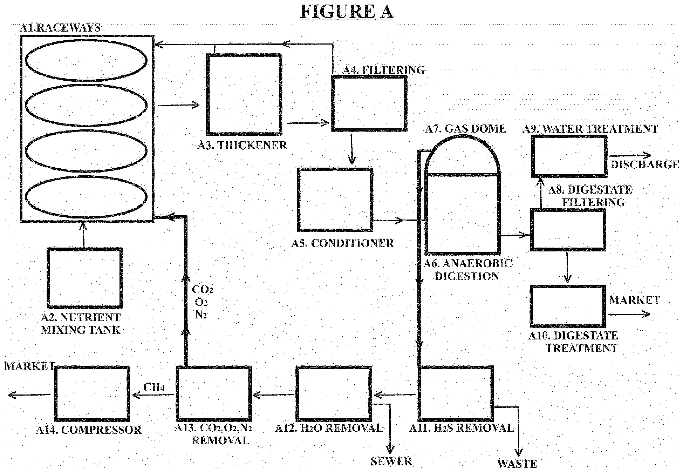

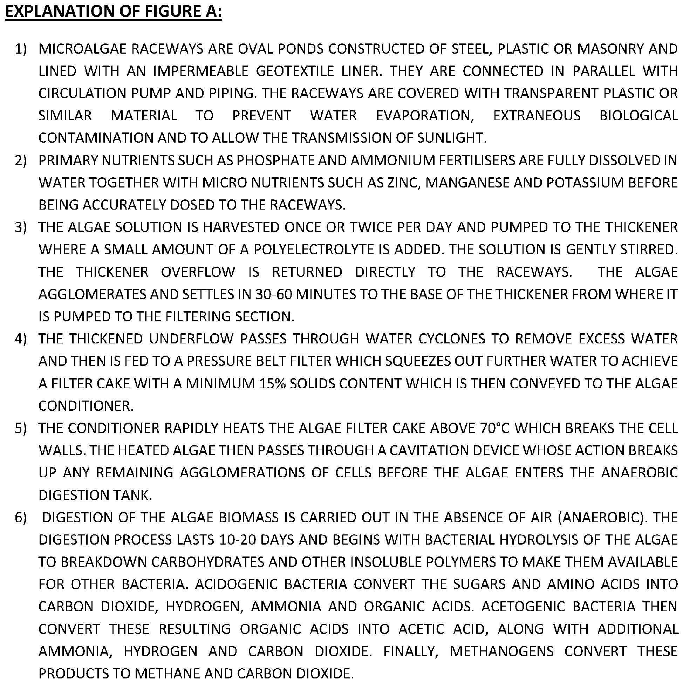

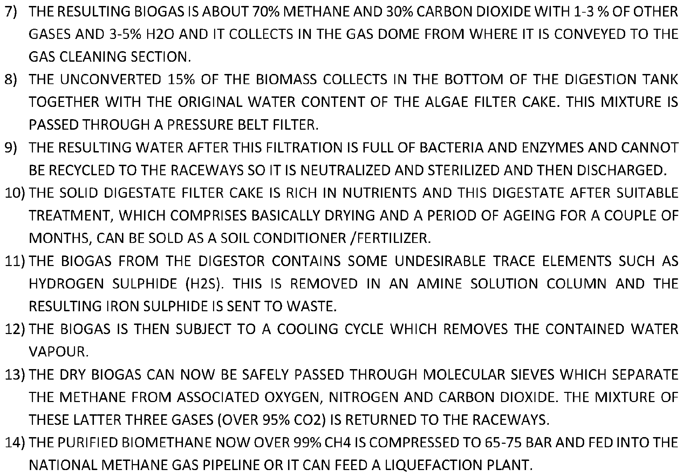

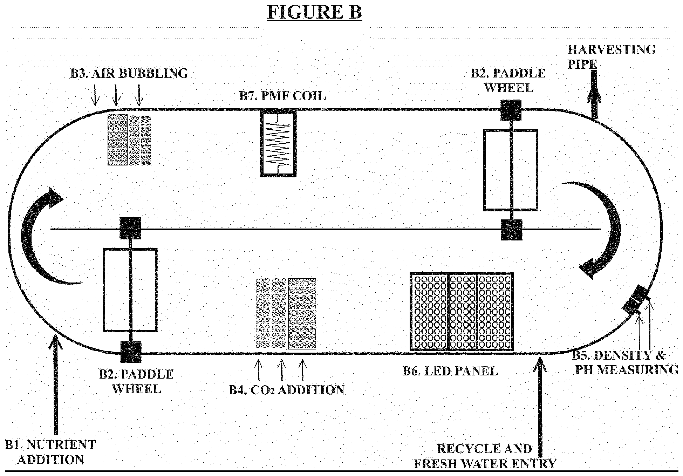

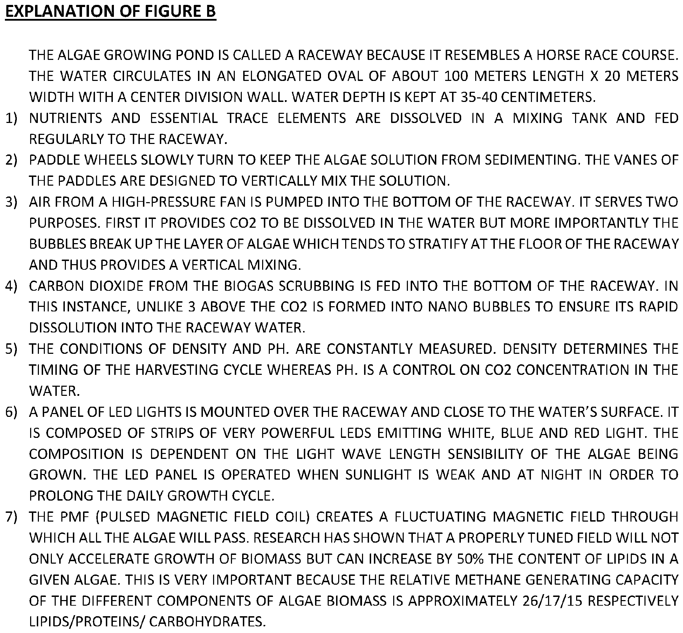

## Claims

1. HIGH YIELD INDUSTRIAL SCALE PRODUCTION OF ALGAL BIOMASS IS ACHIEVED BY APPLYING ALL THE GROWTH PARAMETERS APPLIED IN A LABORATORY SETTING AS WELL AS INTENSIVE MIXING.

2. SPECIFIC WAVELENGTHS OF ARTIFICIAL LIGHT ARE APPLIED AT NIGHT AND LOW SUNLIGHT PERIODS TO LENGTHEN THE DAILY PERIOD OF GROWTH

3. DIFFERENT STRAINS OF A CHOSEN MICROALGAE SPECIES ARE IDENTIFIED WHICH WILL FLOURISH IN THE LOCAL CLIMATIC CONDITIONS

4. CARBON DIOXIDE IS RECYCLED FROM THE BIOGAS SCRUBBERS TO BE FED INTO THE RACEWAYS TO PROMOTE MAXIMUM ALGAE GROWTH

5. PULSED MAGNETIC FIELD (PMF) IS APPLIED TO THE ALGAE TO ACCELERATE GROWTH AND PREFERENTIALLY FAVOR LIPID PRODUCTION. THE PMF PROMOTES A GOOD CELL DENSITY (STIMULATION OF GROWTH AND METABOLIC CASCADES) BY CONTROLLING BIOCHEMICAL PATHWAYS. ALSO, THE PHOTOTROPIC CELLULAR COMMUNICATION IS ENHANCED LEADING TO HIGHER PERCENTAGES OF LIGHT HARVESTING PRIMARY AND ACCESSORY PIGMENTS.
